# EUROPEAN PATENT SPECIFICATION

(11) **EP 3 508 270 B1**
(45) Date of publication and mention of the grant of the patent: **10.03.2021**
(21) Application number: 18382005.9
(22) Date of filing: 09.01.2018
(51) Int. Cl.: B01J 13/14, A61K 38/48, A61K 9/51

(54) **NANOCAPSULE COMPOSITIONS FOR THE CONTROLLED RELEASED OF AGENTS OF THERAPEUTIC INTEREST**
NANOKAPSELZUSAMMENSETZUNGEN ZUR KONTROLLIERTEN FREISETZUNG VON THERAPEUTISCH INTERESSANTEN WIRKSTOFFEN
COMPOSITIONS DE NANOCAPSULE POUR LA LIBÉRATION RÉGULÉE D'AGENTS D'INTÉRÊT THÉRAPEUTIQUE

(43) Date of publication of application: 10.07.2019
(73) Proprietor: Fundación para la Investigación Biomédica del Hospital 12 de Octubre, 28041 Madrid (ES); Universidad Complutense De Madrid, 28040 Madrid (ES)
(72) Inventor: VALLET REGÍ, Maria, 28040 Madrid (ES); VILLEGAS DÍAZ, Maria Rocio, 28040 Madrid (ES); BAEZA GARCÍA, Alejandro, 28040 Madrid (ES); ORTIZ ROMERO, Pablo Luis, 28041 Madrid (ES); PABLOS ALVAREZ, José Luis, 28041 Madrid (ES); USATEGUI CORRAL, Alicia, 28041 Madrid (ES)
(74) Representative: Hoffmann Eitle

(56) References cited:
- WO-A1-2013/033717
- WO-A1-2017/205541
- MARÍA ROCÍO VILLEGAS ET AL: "Hybrid Collagenase Nanocapsules for Enhanced Nanocarrier Penetration in Tumoral Tissues", ACS APPLIED MATERIALS & INTERFACES, vol. 7, no. 43, 21 October 2015 (2015-10-21), pages 24075-24081, XP055515618, US ISSN: 1944-8244, DOI: 10.1021/acsami.5b07116

## Description

### Technical field of the invention

The invention relates to the field of nanocapsule compositions capable of releasing agents of therapeutic interest with a controlled kinetic and a prolonged and sustainable effect over time and uses thereof. The nanocapsule compositions of the present invention are adapted to encapsulate agents of therapeutic interest, namely metal and/or serine proteases, such as collagenase.

### Background of the invention

Fibrosis is a common lesion in different pathological conditions and it is defined by the excessive accumulation of collagen and other extracellular matrix proteins in the connective tissue of different organs where it causes organ dysfunction. Collagen corresponds around of 30% of all proteins in the mammalian bodies, and is the major extracellular protein of different organs such as the skin, cartilage, bones, teeth and cornea. This structural protein is composed by a triple helix of polypeptide strands; one third of the amino acids that composed the molecule are glycine, proline and hydroxyproline. Collagens are synthesized by fibroblasts and other mesenchymal cells and overproduction or inappropriate remodeling and degradation leads to the formation of fibrotic lesions. The process may occur systemically (i.e. systemic sclerosis) or locally (i.e liver cirrhosis) as an abnormal repair process following inflammatory lesions. This can be observed in pathological wound healing, leading to abnormal, hypertrophic or keloid scars or spontaneously, in different diseases such as localized scleroderma (morphea) and other primary local fibrotic lesions that frequently cause local dysfunction (i.e. painful erections in Peyronie disease or hand contractures in Dupuytren disease). Abnormal scars and this type of focal fibrotic lesions can be removed through surgery with variable success. In many cases the process relapses after surgery or is followed by different complications such as deformities and organ dysfunction.

Enzyme-based procedures have been presented as a cost-effective alternative of these types of process because they are minimally invasive techniques that reduce the number and severity of surgical complications and reduce the recovery time.

Collagenase *Clostridium Histolyticum* (CCH) is a zinc-dependent matrix metalloproteinase mixture of AUXI and AUXII collagenases which act synergistically to hydrolyze collagen, which is the primary component of the dense fibrotic cord. The lysis of collagen molecules happens via the peptide bond between repeated sequences of Gly-Pro and a residue X (which often is hydroxyl-proline or proline). AUXI preferentially breaks the collagen fibers close to the N and C-terminal of the collagen triple helical domains, whereas AUXII catalyzes the rupture of collagen from the internal domains. This enzyme shows high specificity so it fails to affect elastic fibers, vascular smooth muscle and axonal myelin sheats. CCH has shown therapeutic effects in a wide number of disorders related with this collagen deposition. Some of the therapeutic applications of this enzyme are related with the treatment of intra-vertebral disc herniation, arterial occlusions, uterine fibroid, degradation of human retained placenta, nipple pain, glaucoma, vitrectomy, burns, abnormal wound healing and keloids, as well as Dupuytren and Peyronie diseases. CCH was approved as an enzymatic treatment by the Food and Drug Administration in USA in February 2010 and by the European Agency for the Evaluation of Medicinal Products (EMEA) since January 2011, and it has been marketed under the name of Xiaflex (Auxilium pharmaceuticals, Inc) for the treatment of Dupuytren and later, in December of 2013, for the treatment of Peyronie disease. The common protocol for the use of Xiaflex in patients with Dupuytren consists of its administration 3 times per cord in intervals of 4 weeks and, in the case of Peyronie, consists of treatment cycles at 6 weeks intervals of two injections every 3 days. These administration rates are due to the labile nature of the enzymes which usually present low chemical and physical stability and suffer from degradation once exposed to the proteases present in the living tissues. Additionally, as a consequence of their short life time in the host, enzyme treatments usually need the injection of dosages higher than those required and therefore, there is an associated risk of side toxicity due to transient local overdose. In the case of CCH injections, these high administration rates and doses produce several problems, as for example, corporal ruptures of penis commonly occurred 5 days after the second injection of each cycle. A decrease in the number of injections and dosages could predictably reduce this associated risk and patient discomfort, increasing tolerability.

In the recent years, nanotechnology has provided interesting strategies for the controlled administration of sensitive therapeutic agents such as proteins, enzymes or even genes, so that their attachment or encapsulation in nanoparticles, or the anchor of polymers on the protein surface of the nanoparticles increases their blood circulation time. Some of these strategies, are based on the attachment of proteins on the surface of nanocarriers, since this approach not only provides to the protein with a greater colloidal stability, but it also provides nanocarriers with novel properties. As an example, the CCH enzyme has been anchored on the surface of different drug delivery nanodevices in order to improve their penetration within solid tumors since these types of malignancies are usually protected by a dense collagen barrier that strongly hampers the nanocarrier difussion. Nanoparticles with proteolytic enzymes such as collagenase or hyaluronidase, among others, immobilized on their surface have demonstrated an increased in the penetration towards the tumor center compared to unmodified nanoparticles. However, these types of systems do not allow a sustained enzyme activity.

In this sense, other approach for the controlled administration of sensitive therapeutic agents such as proteins, enzymes or even genes, is the encapsulation of said sensitive therapeutic agents within polymeric nanocapsules, such as in document WO 2017205541 A1 which provides a nanoscale controlled-release system designed to control the sustained release of a protein cargo in vivo. This strategy has received an increased attention over the last few years. Encapsulating enzymes within a polymeric capsule not only provides protection against external agents present in the host, which is an overriding point, but these polymeric capsules can be engineered in order to release the trapped enzymes in the target place in a controlled manner or even in response to certain stimuli. Villegas *et al.* have recently reported the encapsulation of collagenase within pH-degradable polymeric capsules which released the encapsulated collagenase in 3-10 hours. In this case, and also in the other known methods for collagenase encapsulation, the enzymes are released during a short period of time, in particular during a period lower than 12 hours. In addition, ACS Appl. Mater. Interfaces, 2015, 7, 24075-24081 discloses in Scheme 1, page 24076 and page 24079, polymeric nanocapsules comprising a polymeric shell comprising a polymer derived from a monomer mixture of acrylamide, 2-aminoethyl methacrylate and the crosslinker ethylene glycol dimethylacrylate to encapsulate collagenase. However, although the nanocapsules described in said document maintained the activity during the first 2-3 days, they showed an abrupt decay in the activity afterwards. This limitation hinders the application of these types of nanocapsules for the treatment of diseases which require a sustained enzyme administration during longer times (more than 1 week).

There is thus still a need to provide protease carriers or vehicles capable of achieving a constant and continuous release of the proteases encapsulated within at therapeutic doses during a sufficient period of time for the treatment of diseases which require a sustained protease administration during treatment periods greater than 1 week.

### Summary of the invention

The invention is defined by claims 1 to 10. The present invention solves the above mentioned problems by providing sustained release polymeric nanocapsules capable of releasing metal and/or serine proteases such as collagenase with a controlled kinetic and a prolonged and sustainable effect over time (up to more than 10 days). Additionally, these nanocapsules protect the housed protease, preferably collagenase, against external aggressions maintaining its catalytic activity. In this way, this invention overcomes the currents problems associated with collagenase administration using a precise combination of monomers and cross-linkers, as described in the claims that allow the formation of degradable polymeric nanocapsules with collagenase embedded within. These capsules provide a sustained release of the housed enzyme thus providing a therapeutic improvement in comparison to current therapies thus decreasing the need for repeated injections or high collagenase doses. The efficacy of these capsules has been tested using a murine model of local dermal fibrosis induced by repeated dermal injection of bleomycin, where a higher reduction of fibrosis was observed by using the nanocarriers of the present invention in comparison to the conventional administration of free collagenase.

### Brief description of the figures

**Figure 1****.** Scheme of synthesis of collagenase nanocapsules.
**Figure 2****:** Study of stability of collagenase nanocapsules with different ratios AA/Am. Micrograph by transmission Electron Microscopy a) AA/Am/EG=7.5/6.5/1, b) AA/Am/EG=7/7/1 c) AA/Am/EG=8/6/1 (white scale bar correspond to 100nm) and d) DLS.
**Figure 3****:** Study of stability of collagenase nanocapsules with different ratios EG/MBA. Micrograph by transmission Electron Microscopy a) AA/Am/EG/MBA=7/6/1/1, b) AA/Am/EG/MBA=7/6/1.5/0.5 c) AA/Am/EG/MBA=7/6/0.5/1.5 (white scale bars correspond to 100nm) and d) DLS.
**Figure 4****:** Study of biocompatibility of nanocapsules. Mice were given a fixed amount of nanocapsules or free collagenase. Skin fibrosis was determined by Masson's trichrome staining. Data are representative of one experiment with 5 mice per group. Bar 50µm.
**Figure 5****:** Evaluation of the efficacy of collagenase nanocapsules employing an *in vivo* murine model of skin fibrosis induced by bleomycin injection. C3H mice received daily subcutaneous injections of bleomycin and were treated with free collagenase or nanocapsules. Control mice were daily injected with saline. Fibrosis was measured as the fold-increase in the collagen Masson stained area adjusted to the normal area (saline group). Data are representative of two independent experiments with 10 mice per group. Bar 50µm.

### Detailed description of the invention

In accordance with the present invention, improved biodegradable nanocapsules capable of releasing agents of therapeutic interest with a controlled kinetic and a prolonged and sustainable effect over time are provided. The biodegradable nanocapsules of the present invention are polymeric nanocapsules. The biodegradable nanocapsules of the present invention are adaptable to encapsulate agents of therapeutic interest and deliver the same into *in vivo* upon administration to a recipient. Furthermore, the nanocapsule compositions of the present invention are adaptable for prolonging the sustainable release with a controlled kinetic and a prolonged and sustainable effect over time, of the encapsulated agent contained therein. A novel approach is herein demonstrated, in connection with collagenase to provide a nanocapsule composition having an improved controlled rate of release, *in vivo.*

The present invention thus provides for a novel nanocapsule composition adaptable to encapsulate and effectively deliver agents of therapeutic interest. For example, together with collagenase, the nanocapsule composition may be employed to provide a substitute for the conventional administration of free collagenase. By increasing the controlled rate of release of an encapsulated agent, the nanocapsule composition of the present invention can significantly increase the duration of functioning of the encapsulated agent *in vivo,* thereby enhancing the therapeutic effect of the composition. In addition, the nanocapsule composition of the present invention can effectively deliver a given dosage of a therapeutic agent in a controlled manner, thus improving the effectiveness of the dosage while reducing the overall administration required.

In this sense, and in order to arrive at the present invention, the authors of the invention knew that for the treatment of fibrotic diseases, an efficient Col_{nc} should be engineered in order to provide a sustained release of protease during large periods of time, maintaining unaltered the catalytic activity of the enzyme. For this purpose, a mixture of three types of monomers, acrylamide (AA), 2-aminoethylmethacrylate hydrochloride (Am) and the degradable cross-linker ethyleneglycol dimethacrylate (EG) were studied. The protease:monomer ratio used was 1:2420 and the ratio between monomers was AA/Am/EG 7/6/2. Finally, ammonium persulfate (AP) and *N,N,N,N'-*tetramethylethylenediamine (TMEDA) (1:1.5) were used as radical initiators in a 423:644 ratio with the protease. However, unfortunately by using these conditions, the formed nanocapsules released practically all the collagenase after just merely 10-12 hrs. As it has been extensively mentioned above, this period of time is clearly insufficient for many of the therapeutic applications for which collagenase is currently being used. Therefore, and in order to increase the release time of collagenase, a novel method for the production of collagenase nanocapsules was pursued.

Firstly, the protease:monomer ratio was varied by increasing said ratio, which led to the formation of large aggregates, and a lower ratio was also produced which led to lower nanocapsules formation yields. Therefore, this relation was fixed at the initial protein:monomer ratio, 1:2420.

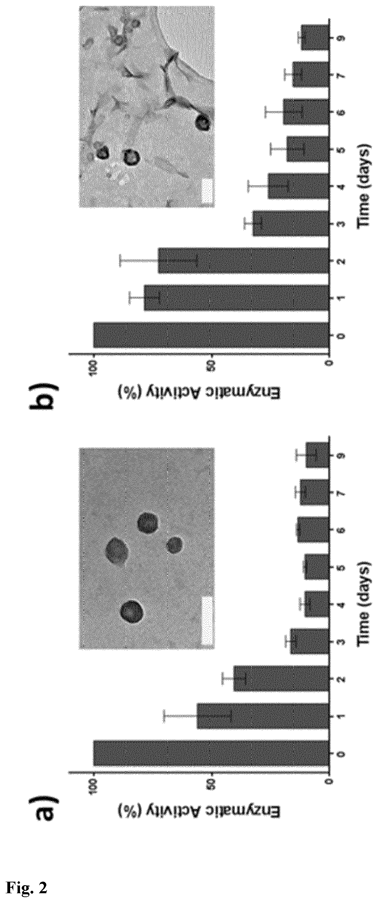

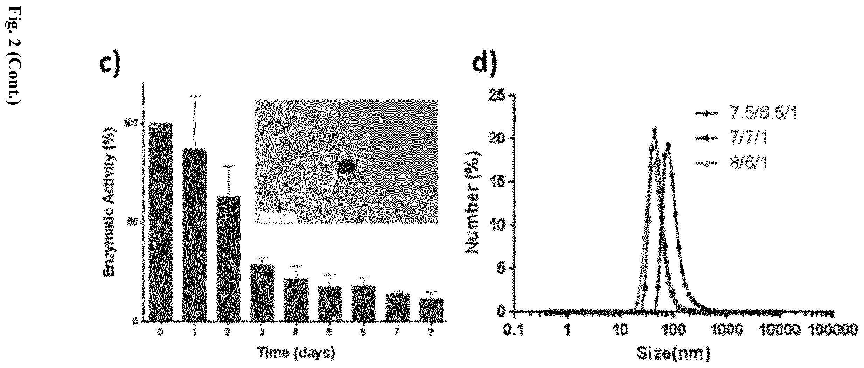

Secondly, the proportions of the degradable cross-linker were varied in order to reduce the hydrolysis kinetic of Col_{nc}. The proportion of this monomer was reduced maintaining constant the total protease monomer ratio which was found optimal. With this strategy, different Col_{nc} were synthesized diminishing the relation of the degradable cross-linker (EG) with the rest of monomers. In all the cases, the results were not completely satisfactory since the effectivity of the protease was maintained only up to three days. When the proportion of the degradable cross-linker were then reduced to half of the initial amount, Col_{nc} were formed as it is shown by dynamic light scattering (DLS) and transmission electron microscopy (TEM) in figure 2, however, these formed nanocapsules lost almost completely their enzymatic activity after merely three days (Figure 2). If the proportion of cross-linker were then reduced to 1/4, Col_{nc} were barely formed because the presence of certain amounts of cross-linker is a compulsory requirement in order to create a homogeneous polymeric shell around the protease.

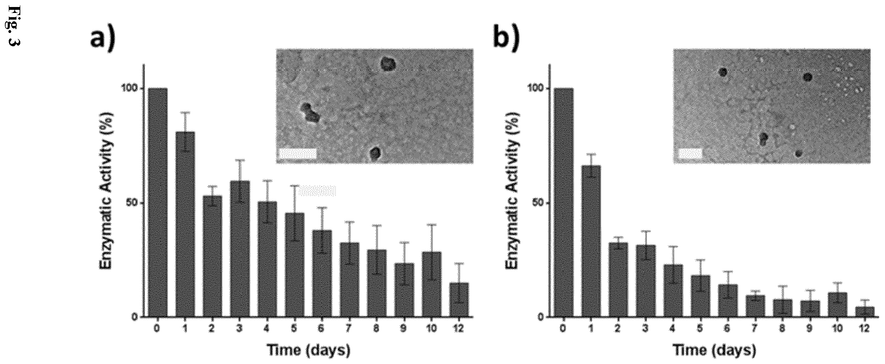

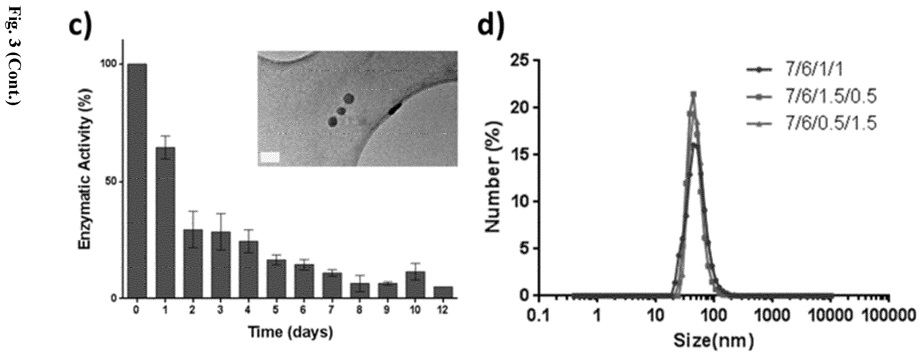

In sight of these results, it was necessary to modify the synthetic approach in order to produce nanocapsules able to release collagenase in a sustained manner for prolonged periods of time. In this sense, the synthetic strategy used consisted in the use of an additional cross-linker in the composition of the capsule; in this case, a non-degradable cross-linker such as bismethacrylamide (MBA). The idea was that the incorporation of this non-degradable cross-linker would allow the formation of the capsule because the ratio of total cross-linker was maintained in the monomer composition and it would increase the degradation time of the capsule achieving a system able to release collagenase during prolonged periods of times. With this in mind, different cross-linker combinations were studied using different percentages of each cross-linker (degradable and non-degradable) keeping constant the protease:monomer ratio at 1:2420 and the AA/Am/cross-linker ratio at 7/6/2. The addition of the new cross-linker did not alter the size and morphology of the resulted Col_{nc}. Among the different Col_{nc} formed with different cross-linker ratios, the nanocapsules formed employing a 1:1 ratio of degradable:non-degradable cross-linkers presented astonishing collagenase release properties as shown in figure 3 (a). These nanocapsules showed a sustained release of collagenase maintaining the activity of the enzyme somewhere close to 50% of the initial one, even after 5 days. Additionally, these nanocapsules presented a sustained enzymatic activity during more than 10 days, maintaining 15% of its activity after 12 days. Unfortunately, the other combinations tested, although they maintained the activity during the first 2-3 days, they showed an abrupt decay in the activity afterwards (see figure 3).

Thus, the Col_{nc} obtained with AA/Am/EG/MBA (7/6/1/1) was chosen as the best system for further clinical evaluation with murine models. Such achieved collagenase released can be highly beneficial in collagenase-based treatments, since the obtained prolonged effect during a prolonged period of time can avoid the necessity to use high doses of the enzyme. In addition, the high stability conferred by this coating composition provides a significant reduction in the administration cycle. Consequently, the nanocapsules of the present invention provide for a promising clinical advance of the current clinical treatment of fibrotic diseases. The effectivity of these nanocapsules was tested in comparison with the current treatment employing a murine fibrotic model as is described in the examples of the present specification.

Therefore, a first aspect of the present invention provides for a polymeric nanocapsule which comprises:
a. A polymeric shell; and
b. one or more metal and/or serine protease encapsulated or embedded in the polymeric shell,
wherein the polymeric shell of a) comprises:
- at least two structural monomers of formula I 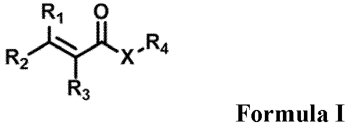 wherein in the first structural monomer R₁, R₂, and R₃, are hydrogen or C₁ to C₄ alkyl groups, X is NH, and R4 is -H;
   wherein in the second structural monomer R₁, R₂, and R₃, are hydrogen or C₁ to C₄ alkyl groups, X is O, and -R₄ is a C₁ to C₄ -alkyl-NH2; and
- at least one degradable and at least one non-degradable cross-linker of Formula II 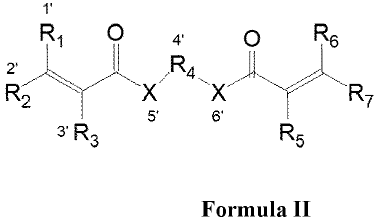 wherein in the degradable cross-linker R_{1 (1')}, R_{2 (2')}, R_{3 (3')}, R₅, R₆ and R₇ are hydrogen or C₁ to C₄ alkyl groups, R_{4 (4')} is a C₁ to C₄ alkyl group, and X _{(5' and 6')} is O; and wherein in the non-degradable cross-linker R_{1 (1')}, R_{2 (2')}, R_{3 (3')}, R₅, R₆ and R₇ a are hydrogen or C₁ to C₄ alkyl groups, R_{4 (4')} is a C₁ to C₄ alkyl group, and X _{(5' and 6')} is NH; and
wherein the protease:monomer ratio and the molar ratio between the first structural monomer/second structural monomer/degradable cross-linker/non-degradable cross-linker are, respectively, 1:2420 and 7:6:1:1.

It is noted that as used herein, the term "nanocapsule" refers to a particle having a core that is surrounded by a shell, such that the particle has a size of less than about 1,000 nanometers. In particular, the nanocapsule of the present invention includes a bioactive component, the bioactive component is located in the core that is surrounded by the shell of the nanocapsule.

It is noted that in the context of the present invention, the term "about" is understood as a deviation of +/- 10%. It is further noted that for all of the ranges mentioned through-out the specification, the limits of the range are included within the range.

In another preferred embodiment of the first aspect of the invention or of any of its preferred embodiments, the metal and/or serine protease is collagenase and/or trypsin.

In another preferred embodiment of the first aspect of the invention or of any of its preferred embodiments, in the degradable cross-linker R_{1 (1')}, R_{2 (2')}, R_{3 (3')}, R₅, R₆ and R₇ are hydrogen and R_{4 (4')} is a C₁ to C₂ alkyl group, and X_{(5' and 6')} is O; and in the non-degradable cross-linker R_{1(1')}, R_{2 (2')}, R_{3 (3')}, R₅, R₆ and R₇ are hydrogen and R_{4 (4')} is a C₁ to C₂ alkyl group, and X_{(5' and 6')} is NH

In another preferred embodiment of the first aspect of the invention or of any of its preferred embodiments, in the second structural monomer R₁, R₂, and R₃, are hydrogen, X is O, and -R₄ is a C₁ to C₂ -alkyl-NH2.

In the present invention, "alkyl groups" is understood to denote in particular a straight chain, branched chain, or cyclic hydrocarbon group usually having from 1 to 20 carbon atoms, preferably having from 1 to 8 carbon atoms, more preferably from 1 to 4 carbon atoms, still more preferably from 1 to 2 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In another preferred embodiment of the first aspect of the invention or of any of its preferred embodiments, the degradable cross-linker is Ethyleneglycol dimethacrylate (EG) and the non-degradable cross-linker is bismethacrylamide (MBA).

In another preferred embodiment of the first aspect of the invention or of any of its preferred embodiments, the first structural monomer is Acrylamide (AA) and the second structural monomer is 2-aminoethylmethacrylate hydrochloride (AM).

In another preferred embodiment of the first aspect of the invention or of any of its preferred embodiments, the degradable cross-linker is Ethyleneglycol dimethacrylate (EG) and the non-degradable cross-linker is bismethacrylamide (MBA), and the first structural monomer is Acrylamide (AA) and the second structural monomer is 2-aminoethylmethacrylate hydrochloride (AM). More preferably, the metal and/or serine protease is collagenase.

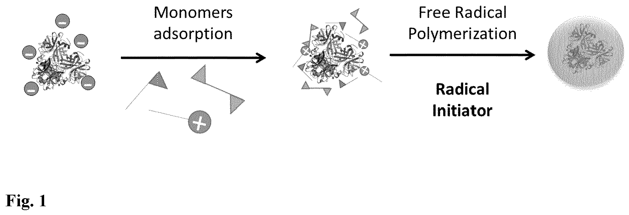

A method for the preparation of the nanocapsule composition of the first aspect of the invention, is exemplified as follows (Figure 1):
1) At first step, the monomers are dissolved in a deoxygenated NaHCO₃ buffer at pH 8.5 in the presence of collagenase. The suspension was stirred at room temperature in an orbital shaker during a certain time in order to provide time for the adsorption of the monomers on the protein surface by electrostatic, hydrogen bonds and Van der Waals interactions.
2) A mixture of radical initiators (ammonium persulfate, APS and *N,N,N',N'-*Tetramethylethylenediamine, TMEDA) was added to the protein-monomer solution at controlled rate in order to start the radical polymerization process. Then, the mixture was stirred at room temperature during 90 min. Collagenase nanocapsules (Col_{nc}) were isolated by centrifugal separation with 10 KDa cut-off filters (AMICON Ultra-2 mL 10KDa).

Therefore, a method is disclosed for obtaining the nanocapsule of the first aspect of the invention or of any of its preferred embodiments, wherein the method comprises:
a) Dissolving the monomers and cross-linkers in the presence of the bioactive molecule;
b) Stirring the suspension a), preferably at room temperature (about 25°C), preferably for about 10 min in order to provide time for the adsorption of the monomers on the biomolecule surface;
c) Adding a mixture of radical initiators, such as for example ammonium persulfate (APS) and N,N,N',N'-Tetramethylethylenediamine (TMEDA), preferably in a molar ratio 1:2 between them and in a molar relation 1:200 to 1:500 protein:APS, preferably about 1:423, in order to start the radical polymerization process;
d) Stirring the mixture; and
e) isolating the nanocapsules.

According to the present invention, a therapeutically effective amount of a metal and/or serine protease, such as collagenase and/or trypsin, is encapsulated in the nanocapsule composition. Therefore the present invention provides a novel delivery system for the controlled release of a therapeutic agent into in vivo circulation.

Therefore, a second aspect of the invention refers to the use of the polymeric nanocapsules of the first aspect of the invention or of any of its preferred embodiments, as a cosmetic of pharmaceutical carrier of active ingredients; preferably, as a cosmetic and/or pharmaceutical carrier of collagenase and/or trypsin activity.

In addition, disclosed but not claimed is where an agent of therapeutic interest, for example, may be encapsulated by the nanocapsule composition of the present invention and introduced into *in vivo* circulation of a recipient by any one of a variety of methods of nanocapsule delivery. For example, a nanocapsule composition of the present invention may be administered by, without limitation, intravenous, intramuscular, intraperitoneal, or subcutaneous delivery, orally or by topical administration, preferably by topical administration. Thus, it is fully contemplated that the nanocapsule composition of the present invention is adaptable to encapsulate virtually any agent of therapeutic interest including macromolecules, in a therapeutically effective concentration, for the purpose of providing a controlled drug delivery system.

Therefore, a third aspect of the invention refers to a pharmaceutical composition comprising the polymeric nanocapsules of the first aspect of the invention or of any of its preferred embodiments, for use in the treatment of skin diseases, preferably via a topical administration, preferably to the skin, using for example collagenase and/or trypsin activity. Preferably, said skin diseases are fibrotic diseases caused by an elevated production of collagen. More preferably, such pharmaceutical composition is used for the treatment of intra-vertebral disc herniaton, arterial occlusions, uterine fibroid, degradation of human retained placenta, nipple pain, glaucoma, vitrectomy, burns, abnormal wound healing and keloids, as well as Dupuytren and Peyronie diseases.

Lastly, a fourth aspect of the invention refers to a cosmetic composition comprising the polymeric nanocapsules of the first aspect of the invention or of any of its preferred embodiments. Preferably, such cosmetic composition is use as a vehicle or carrier of bioactive molecules such as metal and/or serine protease, such as collagenase and/or trypsin activity to the skin. More preferably, such cosmetic composition is use, preferably via a topical administration, in a subject or recipient suffering from fibrotic diseases caused by an elevated production of collagen. More preferably, such subject or recipient suffers from of intra-vertebral disc herniaton, arterial occlusions, uterine fibroid, degradation of human retained placenta, nipple pain, glaucoma, vitrectomy, burns, abnormal wound healing and keloids, as well as Dupuytren and Peyronie diseases.

The present invention will be more readily understood by referring to the following examples which are given to illustrate the invention rather than to limit its scope.

### Examples

### Example 1. Evaluation in mouse models of the nanocapsules of the invention

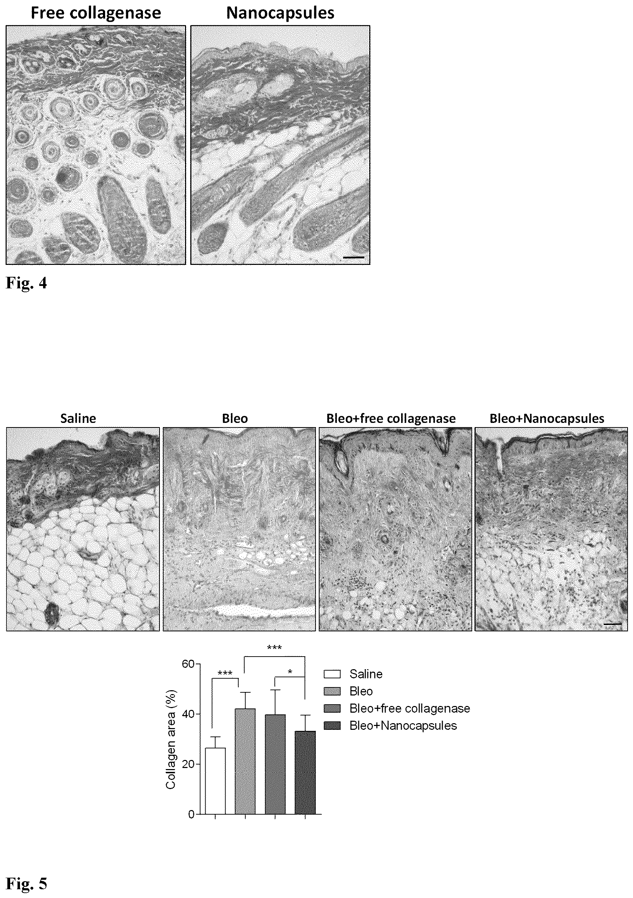

The biocompatibility of the collagenase nanocapsules as described in figure 3 (a) were tested using an *in vivo* model based in healthy mice. With this purpose, the same amount of enzyme (300 micrograms) encapsulated and free, was injected subcutaneously in healthy mice. After 10 days, the animals were sacrificed and the absence of local toxicity was observed in the histological studies (Figure 4). In order to analyze the therapeutic efficacy of collagenase nanocapsules *in vivo,* a bleomycin-induced animal model of dermal fibrosis was used. Skin fibrosis was induced in 6-week-old female C3H/HeNHsd mice by subcutaneous daily injection of 100 micrograms of bleomycin (1mg / ml) or saline (0.9%) in a skin-shaved area of the back every day by subcutaneous injections for 4 weeks. Upon that time, 300 micrograms of the encapsulated nanocapsules as described in figure 3 (a) or free collagenase were injected subcutaneously and maintained for 10 days. Fibrosis was evaluated as the increase in the collagen-stained area on Masson's trichrome stained skin sections. The administration of a single dose of collagenase nanocapsules produced a significant decrease of the collagen area in the affected area compared to the administration of the same dose of free collagenase (Figure 5).

## Claims

1. A polymeric nanocapsule which comprises:
a. A polymeric shell; and
b. one or more metal and/or serine proteases encapsulated or embedded in the polymeric shell, wherein the polymeric shell of a) comprises:
- at least two structural monomers of formula I: 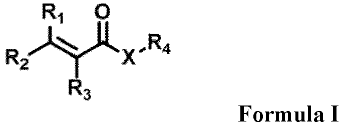 wherein in the first structural monomer R₁, R₂, and R₃, are hydrogen or C₁ to C₄ alkyl groups, X is NH, and R4 is -H;
wherein in the second structural monomer R₁, R₂, and R₃, are hydrogen or C₁ to C₄ alkyl groups, X is O, and -R₄ is a C₁ to C₄ -alkyl-NH2; and
- at least one degradable and at least one non-degradable cross-linker of Formula II: 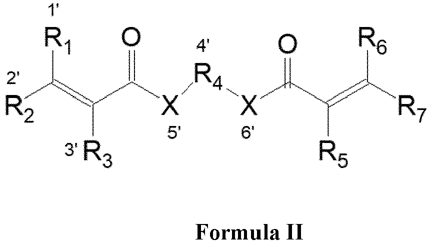 wherein in the degradable cross-linker R_{1 (1')}, R_{2 (2')}, R_{3 (3')}, R₅, R₆ and R₇ a are hydrogen or C₁ to C₄ alkyl groups, R_{4 (4')} is a C₁ to C₄ alkyl group, and X _{(5' and 6')} is O; and wherein in the non-degradable cross-linker R_{1 (1')}, R_{2 (2')}, R_{3 (3')}, R₅, R₆ and R₇ a are hydrogen or C₁ to C₄ alkyl groups, R_{4 (4')} is a C₁ to C₄ alkyl group, and X _{(5' and 6')} is NH; and
wherein the protease:monomer ratio and the molar ratio between the first structural monomer/second structural monomer/degradable cross-linker/non-degradable cross-linker are, respectively, 1:2420 and 7:6:1:1.

2. The polymeric nanocapsule according to claim 1, wherein the metal and/or serine protease is collagenase and/or trypsin.

3. The polymeric nanocapsule, according to claim 1, wherein the metal and/or serine protease is collagenase.

4. The polymeric nanocapsule, according to any of claims 1 to 3, wherein in the degradable cross-linker R_{1 (1')}, R_{2 (2')}, R_{3 (3')}, R₅, R₆ and R₇ a are hydrogen and R_{4 (4')} is a C₁ to C₂ alkyl group, and X _{(5' and 6')} is O; and wherein in the non-degradable cross-linker R_{1 (1')}, R_{2 (2')}, R_{3 (3')}, R₅, R₆ and R₇ are hydrogen and R_{4 (4')} is a C₁ to C₂ alkyl group, and X _{(5' and 6')} is NH.

5. The polymeric nanocapsule, according to any of claims 1 to 4, wherein in the second structural monomer R₁, R₂, and R₃, are hydrogen, X is O, and -R₄ is a C₁ to C₂ -alkyl-NH2.

6. The polymeric nanocapsule according to any of claims 1 to 5, wherein the degradable cross-linker is Ethyleneglycol dimethacrylate (EG) and the non-degradable cross-linker is bismethacrylamide (MBA).

7. The polymeric nanocapsule according to any of claims 1 to 5, wherein the first structural monomer is Acrylamide (AA) and the second structural monomer is 2-aminoethylmethacrylate hydrochloride (AM).

8. The polymeric nanocapsule according to any of claims 1 to 5, wherein the degradable cross-linker is Ethyleneglycol dimethacrylate (EG) and the non-degradable cross-linker is bismethacrylamide (MBA), and wherein the first structural monomer is Acrylamide (AA) and the second structural monomer is 2-aminoethylmethacrylate hydrochloride (AM).

9. The polymeric nanocapsule according to claim 8, wherein the bioactive molecule is collagenase.

10. Use of the polymeric nanocapsules of any of claims 1 to 9, as a cosmetic of pharmaceutical carrier of active ingredients.

## Patentansprüche

1. Polymere Nanokapsel, welche Folgendes umfasst:
a. eine polymere Hülle; und
b. eine oder mehrere Metall- und/oder Serinproteasen, welche in der polymeren Hülle eingekapselt oder eingebettet sind,
wobei die polymere Hülle aus a) Folgendes umfasst:
- mindestens zwei strukturelle Monomere der Formel I: 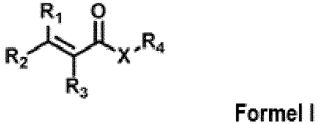 wobei im ersten strukturellen Monomer R₁, R₂ und R₃ Wasserstoff oder C₁- bis C₄-Alkylgruppen sind, X NH ist und R₄ -H ist;
wobei im zweiten strukturellen Monomer R₁, R₂ und R₃ Wasserstoff oder C₁- bis C₄-Alkylgruppen sind, X O ist und -R₄ ein C₁- bis C₄ -Alkyl-NH2 ist; und
- mindestens einen abbaubaren und mindestens einen nicht abbaubaren Vernetzer der Formel II: 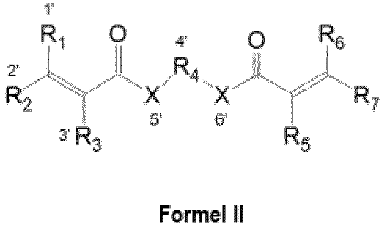 wobei im abbaubaren Vernetzer R_{1 (1')}, R_{2 (2')}, R_{3 (3')}, R₅, R₆ und R₇ Wasserstoff oder C₁- bis C₄-Alkylgruppen sind, R_{4 (4')} eine C₁- bis C₄-Alkylgruppe ist und X _{(5' und 6')} O ist; und wobei im nicht abbaubaren Vernetzer R_{1 (1')}, R_{2 (2')}, R_{3 (3')}, R₅, R₆ und R₇ Wasserstoff oder C₁- bis C₄-Alkylgruppen sind, R_{4 (4')} eine C₁- bis C4-Alkylgeruppe ist und X _{(5' und 6')} NH ist; und
wobei das Protease:Monomer-Verhältnis und das Molverhältnis zwischen dem ersten strukturellen Monomer/zweiten strukturellen Monomer/abbaubaren Vernetzer/nicht abbaubaren Vernetzer jeweils 1:2420 und 7:6:1:1 sind.

2. Polymere Nanokapsel nach Anspruch 1, wobei die Metall- und/oder Serinprotease Collagenase und/oder Trypsin ist.

3. Polymere Nanokapsel nach Anspruch 1, wobei die Metall- und/oder Serinprotease Collagenase ist.

4. Polymere Nanokapsel nach einem der Ansprüche 1 bis 3, wobei im abbaubaren Vernetzer R_{1 (1')}, R_{2 (2')}, R_{3 (3')}, R₅, R₆ und R₇ Wasserstoff sind und R_{4 (4')} eine C₁- bis C₂-Alkylgruppe ist und X _{(5' und 6')} O ist; und wobei im nicht abbaubaren Vernetzer R_{1 (1')}, R_{2 (2')}, R_{3 (3')}, R₅, R₆ und R₇ Wasserstoff sind und R_{4 (4')} eine C₁- bis C₂-Alkylgruppe ist und X _{(5' und 6')} NH ist.

5. Polymere Nanokapsel nach einem der Ansprüche 1 bis 4, wobei im zweiten strukturellen Monomer R₁, R₂ und R₃ Wasserstoff sind, X O ist und -R₄ ein C₁- bis C₂-Alkyl-NH2 ist.

6. Polymere Nanokapsel nach einem der Ansprüche 1 bis 5, wobei der abbaubare Vernetzer Ethylenglycoldimethacrylat (EG) ist und der nicht abbaubare Vernetzer Bismethacrylamid (MBA) ist.

7. Polymere Nanokapsel nach einem der Ansprüche 1 bis 5, wobei das erste strukturelle Monomer Acrylamid (AA) ist und das zweite strukturelle Monomer 2-Aminoethylmethacrylathydrochlorid (AM) ist.

8. Polymere Nanokapsel nach einem der Ansprüche 1 bis 5, wobei der abbaubare Vernetzer Ethylenglycoldimethacrylat (EG) ist und der nicht abbaubare Vernetzer Bismethacrylamid (MBA) ist und wobei das erste strukturelle Monomer Acrylamid (AA) ist und das zweite strukturelle Monomer 2-Aminoethylmethacrylathydrochlorid (AM) ist.

9. Polymere Nanokapsel nach Anspruch 8, wobei das bioaktive Molekül Collagenase ist.

10. Verwendung der polymeren Nanokapseln nach einem der Ansprüche 1 bis 9, als kosmetischer oder pharmazeutischer Träger von Wirkstoffen.

## Revendications

1. Nanocapsule polymérique qui comprend :
a. une coque polymérique; et
b. une ou plusieurs protéases métalliques et/ou serine-protéases encapsulées ou intégrées dans la coque polymérique,
dans laquelle la coque polymérique de a) comprend :
- au moins deux monomères structuraux de formule I : 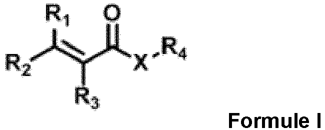 dans laquelle dans le premier monomère structural R₁, R₂ et R₃ sont de l'hydrogène ou des groupes alkyle C₁ à C₄, X est NH et R₄ est -H ;
dans laquelle dans le second monomère structural R₁, R₂ et R₃ sont de l'hydrogène ou des groupes alkyle C₁ à C₄, X est O et -R₄ est un -alkyle-NH₂ C₁ à C₄; et
- au moins un agent de réticulation dégradable et au moins un agent de réticulation non dégradable de formule II : 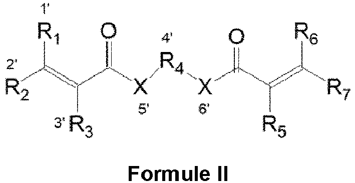 dans laquelle dans l'agent de réticulation dégradable R_{1(1')}, R_{2(2')}, R_{3(3')}, R₅, R₆ et R₇ sont de l'hydrogène ou des groupes alkyle C₁ à C₄, R_{4(4')} est un groupe alkyle C₁ à C₄, et X _{(5' et 6')} est O; et dans laquelle dans l'agent de réticulation non dégradable R_{1(1')}, R_{2(2')}, R_{3(3')}, R₅, R₆ et R₇ sont de l'hydrogène ou des groupes alkyle C₁ à C₄, R_{4(4')} est un groupe alkyle C₁ à C₄, et X_{(5' et 6')} est NH ; et
dans laquelle le rapport protéase:monomère et le rapport molaire entre le premier monomère structural/second monomère structural/agent de réticulation dégradable/agent de réticulation non dégradable sont, respectivement, 1:2420 et 7:6:1:1.

2. Nanocapsule polymérique, selon la revendication 1, dans laquelle la protéase métallique et/ou la sérine-protéase est de la collagénase et/ou de la trypsine.

3. Nanocapsule polymérique, selon la revendication 1, dans laquelle la protéase métallique et/ou la sérine-protéase est de la collagénase.

4. Nanocapsule polymérique, selon l'une quelconque des revendications 1 à 3, dans laquelle dans l'agent de réticulation dégradable R_{1(1')}, R_{2(2')}, R_{3(3')}, R₅, R₆ et R₇ sont de l'hydrogène et R_{4 (4')} est un groupe alkyle C₁ à C₂, et X _{(5' et 6')} est O ; et dans laquelle dans l'agent de réticulation non dégradable R_{1(1')}, R_{2(2')}, R_{3(3')}, R₅, R₆ et R₇ sont de l'hydrogène et R_{4(4')} est un groupe alkyle C₁ à C₂, et X_{(5' et 6')} est NH.

5. Nanocapsule polymérique, selon l'une quelconque des revendications 1 à 4, dans laquelle dans le second monomère structural R₁, R₂ et R₃ sont de l'hydrogène, X est O et -R₄ est un alkyle-NH2 C₁ à C₂.

6. Nanocapsule polymérique, selon l'une quelconque des revendications 1 à 5, dans laquelle l'agent de réticulation dégradable est du dimétachrytate d'éthylèneglycol (EG) et l'agent de réticulation non dégradable est de la bisméthacrylamide (MBA).

7. Nanocapsule polymérique, selon l'une quelconque des revendications 1 à 5, dans laquelle le premier monomère structural est de l'acrylamide (AA) et le second monomère structural est de l'hydrochlorure du méthacrylate de 2-aminoéthyle (AM).

8. Nanocapsule polymérique, selon l'une quelconque des revendications 1 à 5, dans laquelle l'agent de réticulation dégradable es du dimétachrylate d'éthylèneglycol (EG) et l'agent de réticulation non dégradable est de la bisméthacrylamide (MBA), et dans laquelle le premier monomère structural est de l'acrylamide (AA) et le second monomère structural est de l'hydrochlorure du méthacrylate de 2-aminoéthyle (AM).

9. Nanocapsule polymérique, selon la revendication 8, dans laquelle la molécule bioactive est de la collagénase.

10. Utilisation des nanocapsules polymériques selon l'une quelconque des revendications 1 à 9, sous forme de support cosmétique ou pharmaceutique d'ingrédients actifs.
